# EUROPÄISCHE PATENTSCHRIFT

(11) **EP 1 843 723 B1**
(45) Veröffentlichungstag und Bekanntmachung des Hinweises auf die Patenterteilung: **03.03.2010**
(21) Anmeldenummer: 05707733.1
(22) Anmeldetag: 09.03.2005
(51) Int. Cl.: A61F 2/44

(54) **AUFSPREIZBARES IMPLANTAT ZUR ANORDNUNG ZWISCHEN DEN WIRBELKÖRPERN**
EXPANDABLE IMPLANT PLACEABLE BETWEEN TWO VERTEBRAL BODIES
IMPLANT EXPANSIBLE DESTINE A ETRE PLACE ENTRE DES CORPS VERTEBRAUX

(30) Priorität: 01.02.2005 DE 102005004563
(43) Veröffentlichungstag der Anmeldung: 17.10.2007
(73) Patentinhaber: Advanced Medical Technologies AG, 66620 Nonnweiler-Braunshausen (DE)
(72) Erfinder: WAWRO, Wolfgang, 06193 Gutenberg (DE); HOELL, Thomas, 06193 Gutenberg (DE); WEILAND, Peter, 66620 Nonnweiler (DE)
(74) Vertreter: Kruspig, Volkmar
(86) Internationale Anmeldenummer: PCT/EP2005/002501
(87) Internationale Veröffentlichungsnummer: WO 2006/081843

(56) Entgegenhaltungen:
- DE-A1- 10 248 170
- US-A- 6 129 763
- US-B1- 6 190 414
- US-B1- 6 814 756

## Beschreibung

Die Erfindung betrifft ein aufspreizbare Implantat zur vorzugsweise dorsalen Anordnung zwischen den Wirbelkörpern der Wirbelsäule zum Zweck, diese mechanisch zu verbinden und eine knöcherne Durchbauung zu unterstützen, bestehend aus zwei, an einem Ende miteinander verbundenen Schenkeln, welche eine Keilform bilden sowie mit einem, zwischen den Schenkeln angeordneten, insbesondere verschiebbaren Spreizelement zur vertikalen Distraktion der Schenkel gemäß Oberbegriff des Patentanspruches 1.

Implantate zum Ersatz von Wirbelkörpern und/oder zur Stabilisierung und Fixierung der Wirbelsäule gehören seit längerem zum Stand der Technik.

Bekannt sind beispielsweise Wirbelkörperplatzhalter gemäß DE 296 16 778 U1, welche eine Hülsenform aufweisen, wobei die Stirnenden der Hülse eine unregelmäßige Kante bilden und wobei die Wandung der Hülsen Löcher zur Aufnahme von Knochensubstanz aufweisen. Bei diesen bekannten Wirbelkörperplatzhaltern verläuft die vorerwähnte unregelmäßige Kante an mindestens einem Stirnende in einem Winkel zur Querachse des hülsenförmigen Körpers, um hier eine Anpassung an die anatomischen Gegebenheiten zu bewirken. Durch die Anordnung von zwei, ineinander geführten, höhenverstellbaren Hülsen besteht die Möglichkeit der Aufspreizung zum Erhalt einer entsprechenden Anlage an den jeweiligen Wirbelkörperflächen.

Bezüglich der Höhenverstellbarkeit finden sich im Stand der Technik Lösungen, die auch U-förmig ineinander geführte Implantate umfassen, welche mit Hilfe eines speziellen Aufspreizinstrumentes von einer zusammengeschobenen Position in eine entsprechende Endlageposition überführt werden können. Beispielsweise sei hier auf die US-PS 5,290,312 oder die DE 44 09 392 A1 verwiesen.

Bei dem gattungsbildenden Spreizimplantat zur Anordnung zwischen Wirbeln der Wirbelsäule gemäß DE 102 48 170 A1 umfasst dieses zwei an einem Ende miteinander verbundene, jeweils gegen einen der Wirbel anlegbare Schenkel und eine Einrichtung zur vertikalen Distraktion der Schenkel.
Gemäß DE 102 49 170 A1 konvergieren in der Ausgangsstellung für die Distraktion die den Wirbeln zugewandten Außenseiten der Schenkel zu den freien Schenkelenden hin. Durch diese Keilform soll die Einführung des Implantates in den Zwischenraum zwischen den Wirbeln erleichtert werden. Mit Hilfe des vorbekannten Spreizelementes bei dem erwähnten Implantat besteht die Möglichkeit, durch translatorische Bewegung die Schenkel des Implantates aufzuspreizen, so dass diese im aufgespreizten Zustand über ihre gesamte horizontale Breite abgestützt werden. An einer den freien Schenkelenden gegenüberliegenden Seite besitzt das Implantat nach DE 102 48 170 A1 eine Öffnung für die Durchführung eines Werkzeuges mit dessen Hilfe der Spreizschieber bewegt werden kann.

Durch biologische Abnutzung und angeborene Fehlbildungen kann es bekanntermaßen zu Veränderungen der Bandscheiben, der Knochen sowie der die Wirbelkörper verbindenden Gelenke kommen. In der Summe resultiert daraus üblicherweise eine Verschiebung der Wirbelkörper gegeneinander oder eine Verdrehung bzw. Verkippung.
Dies führt häufig dazu, dass die Verbindungselemente, die Wirbelgelenke falsch belastet werden und der Körper Bindegewebe anlagert. Dieser Effekt sowie der direkte mechanische Einfluss verschobener Wirbelkörper können zu einer Kompression von Nerven und damit zu einer Schädigung und Lähmungen bzw. sensiblen Ausfällen führen.

Um diese degenerativen Veränderungen auszugleichen und die Wirbelkörper in einer anatomisch günstigen Stellung zueinander zu fixieren, wurden bestimmte operative Techniken entwickelt. Entweder wird von dorsal durch den Rücken oder von ventral durch den Bauch die Bandscheibe entfernt und diese durch ein geeignetes technisches Interponat ersetzt. Gleichzeitig wird dabei die Wirbelsäule in geeigneter Weise ausgerichtet, so dass die empfindlichen Nerven nicht mehr durch knöcherne oder bindegewebige Anteile unerwünscht komprimiert werden. Um die notwendige Stabilität zu erreichen, wird anstelle der Bandscheibe ein Implantat eingesetzt. Diese Implantate können von vorne, von der Seite oder aber auch von hinten implantiert werden.

Bekannte, nicht höhenvariable Implantate sind räumliche Gebilde, die überwiegend eine Hohlform aufweisen. Die Implantate werden operativ mit Knochenmaterial oder Knochenersatzmaterial befüllt und wie oben erwähnt nach Entfernung von Bandscheibenmaterial zwischen die Wirbelkörper eingefügt.

Eines der wesentlichen Probleme bei der Implantation von Implantaten vorbei an den Nervenwurzeln in den Bandscheibenraum ist die Tatsache, dass die Implantate relativ groß sind, hingegen der Platz zwischen dem Duralsack und den abgehenden Nervenwurzeln nur geringe Abmessungen aufweist. Aus diesem Grunde ergibt sich eine Größenbeschränkung für Implantate mit Blick auf die jeweilige zu operierende Höhe.

Um dieses Problem zu überwinden, wurde das bereits gewürdigte Implantat nach DE 102 48 170 A1 entwickelt. Dieses Implantat lässt sich im Bandscheibenraum auf die notwendige Größe aufspreizen und ist im nichtaufgespreizten Zustand leicht zu handhaben.

Bei der verletzten Wirbelsäule ist eines der häufigsten Verletzungsmuster derart ausgeprägt, dass die Vorderkante der Wirbelkörper durch Sturz oder Schlag zusammengedrückt wird, wodurch ein Keilwinkel entsteht.

Hieraus resultiert, dass ein Implantat, welches eine kraftschlüssige Verbindung zwischen zwei Wirbelkörpern herstellen soll, diesen Keilwinkel ausgleichen muss, so dass seine Ober- und Unterseite die jeweilige Unter- oder Oberseite der Wirbelkörper berührt.

Bei Traumen ist im Gegensatz zu degenerativen Veränderungen der Wirbelsäule dieser Keilwinkel besonders stark ausgeprägt.
Das heißt, die notwendige Größe im bauchseitigen Abschnitt des Wirbelkörpers liegt deutlich über derjenigen, die man üblicherweise aus anatomischen bzw. technischen Gründen von dorsal her einbringen kann.

Dieses bekannte Problem wurde bisher dadurch umgangen, dass entsprechend vorgeformte Implantate mit derart großen Keilwinkeln von der Bauchseite her in die Wirbelsäule eingefügt wurden.

Der Zugang über die Flanke (retroperitoneal) oder über die Vorderseite der Wirbelsäule (transperitoneal) durch den Bauch birgt aber besondere Risiken, die zu einer recht hohen operationsbedingten Erkrankungsrate führen. Dieses Risiko kann durch eine dorsale Versorgung deutlich reduziert werden. Bisher wurden jedoch keine technischen Lösungen bekannt, die es ermöglichen, ein großes und gewinkeltes Implantat vom Rücken statt von der Bandscheibe hier einzusetzen.

Zusammenfassend verbleibt festzustellen, dass die bisherigen Fusionsverfahren für Wirbelsäulen, die vom Bauch her oder von der Flanke her durchgeführt werden, ein relativ hohes Operationsrisiko tragen. Es bedarf hier eines Chirurgen, der sowohl qualifiziert ist, an der Wirbelsäule zu operieren als auch im Bauch bzw. im Retroperitonium. Eine solche Qualifikation ist jedoch seltener anzufinden, als eine Qualifikation des Chirurgen, allein an der Wirbelsäule operieren zu dürfen.

Implantate, die durch den Bauch oder durch die Flanke eingesetzt werden, sind relativ groß im Verhältnis zu den Operatiönszugängen, so dass das Operieren sehr schwierig und die Operationszeiten lang und die Möglichkeiten für Fehlplatzierungen oder suboptimale Ergebnisse hoch sind.

Ein aufspreizbares Implantat gemäß dem Oberbegriff von Anspruch 1 ist in DE 102 48 170 A1 offenbart.

Ausgehend hiervon ist "die" Aufgabe der Erfindung das Operationsrisiko beim Einführen des Implantats in die Wirbelsäule zu verringern.

Das erfindungsgemäße Merkmal des Implantates besteht in der kraft- und/oder formschlüssigen, lösbaren Arretierung, welche im Bereich der freien Schenkelenden zum Fixieren der Schenkel in einer möglichst eng aneinanderliegenden Position vorgesehen ist.

Hierdurch weist das Implantat nur eine geringe Größe auf, wenn es inseriert wird, so dass es dem Operateur in einfacher Weise möglich ist, mit dem Implantat umzugehen. Vergleichsweise verhält sich im zusammengehaltenen Zustand das Implantat wie ein steifes, nicht aufspreizbares Objekt.

Durch das Zusammenführen der Schenkel an der Vorderseite des Implantates und die Fixierung in diesem Zustand durch die Arretierungsmittel wird demnach das Implantat so klein gebaut, dass es zwischen den hinteren Ringapophysen hindurch implantiert werden kann. Ein weiterer wesentlicher Vorteil liegt darin, dass durch die enge Zusammenführung der beiden Schenkel die Gefahr verringert ist, umliegendes Gewebe, insbesondere Nervengewebe mit in den Bandscheibenraum hineinzuziehen. Hierdurch wird die Implantation insgesamt sicherer.

In bevorzugter Ausgestaltung der Erfindung ist das Vorderende des Implantates stark abgerundet oder spitz zulaufend, was sowohl die Implantation in kleine Zwischenwirbelräume erleichtert als auch gleichzeitig eine optimale Anpassung an die ventrale Ringapophyse erlaubt. Die erwähnte Form stellt ebenso einen Reservoirraum für die Implantation von Spongiosa bereit.

Zusammenfassend bleibt die durch die Schenkel gebildete Keilform des Implantates auch nach der Distraktion bezüglich des Keilwinkels mindestens erhalten oder unterliegt einer gezielten Vergrößerung, wobei das Maß der Aufspreizung durch das Spreizelement in Verbindung mit einer konvergierenden Führung an mindestens einer der Innenseiten der Schenkel bestimmt wird. Über die Ausbildung der konvergierenden Führung an mindestens einer der Innenseiten der Schenkel ist in Verbindung mit dem verschiebbaren Spreizelement der End-Keilwinkel, den die Schenkel des Implantates im voll aufgespreizten Zustand einnehmen, einstell- bzw. vorgebbar.

Wie bereits erwähnt, ist im Bereich der freien Schenkelenden zum Fixieren der Schenkel in einer möglichst eng aneinanderliegenden Position vorgesehen, kraft- und/oder formschlüssige lösbare Arretierungsmittel anzuordnen.
Diese Arretierungsmittel können eine Snap-in-Verbindung bilden.

Diese Snap-in-Verbindung ist beispielsweise so ausführbar, dass an den gegenüberliegenden Flächen der freien Schenkelenden einerseits eine Rastnase sowie andererseits eine komplementär ausgebildete Rastnut vorgesehen wird.

Beim Verschieben des Spreizelementes in Distraktionsrichtung löst sich der Form- und/oder Kraftschluss zwischen den freien Schenkelenden.

Das Spreizelement ist ausgestaltend als sogenannter Spreizblock ausgebildet, welcher ausschließlich eine Bewegung in eine zu Distraktion senkrechte Richtung ausführt.

Weiterhin weist das Implantat Nuten oder Bohrungen oder ähnlich gestaltete Ausnehmungen für Röntgenkontraststifte auf. Diese Maßnahme ist insbesondere dann von Vorteil, wenn das Implantat aus einem bioelastischen Kunststoffmaterial, beispielsweise PEEK besteht.

Das vorerwähnte Kunststoffmaterial verfügt über eine Restelastizität, so dass eine dynamische Anpassung an die Mikrobewegung der Wirbelendplatten möglich wird.

Die Ober- und Unterseiten der Schenkel besitzen an sich bekannte Durchbrüche zur Aufnahme von Knochensubstanz zum Zweck der gewünschten Fusion.

Mit dem vorgestellten Implantat ist eine optimale Versorgung mit guter Unterfütterung der Wirbel und guter postoperativer Primärstabilität erreichbar. Im übrigen treten die meisten Frakturen zwischen Th12 und L2 auf. Hier ist es für nicht spreizbare Implantate derartig eng, dass eine Operation mit vertretbarem Risiko kaum möglich ist. Erst mit dem vorgestellten Implantat besteht die Möglichkeit, Operationen in den entscheidenden Höhen bei akzeptablem Risiko zu realisieren.

Die Erfindung soll nachstehend anhand eines Ausführungsbeispieles sowie unter Zuhilfenahme von Figuren näher erläutert werden.

Hierbei zeigen:
- Figur 1: eine Seitenansicht des erfindungsgemäßen Implantates;

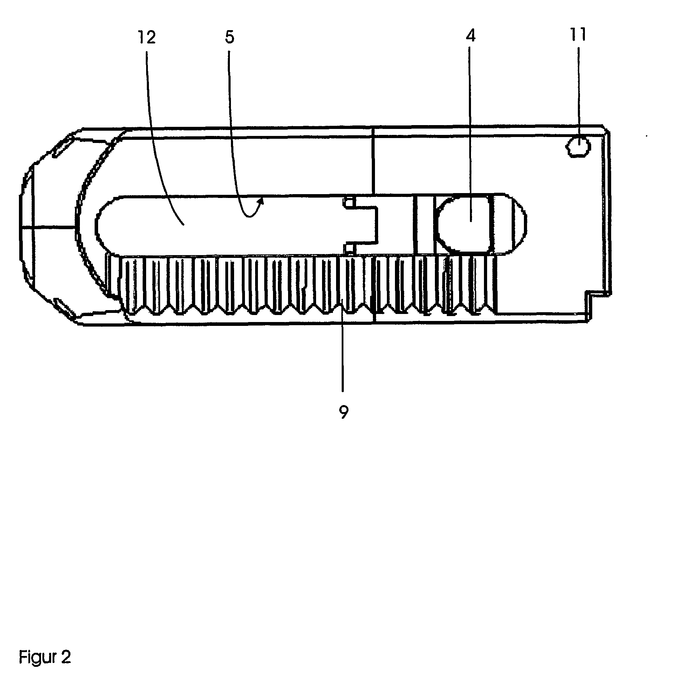

- Figur 2: eine Draufsicht des Implantates;

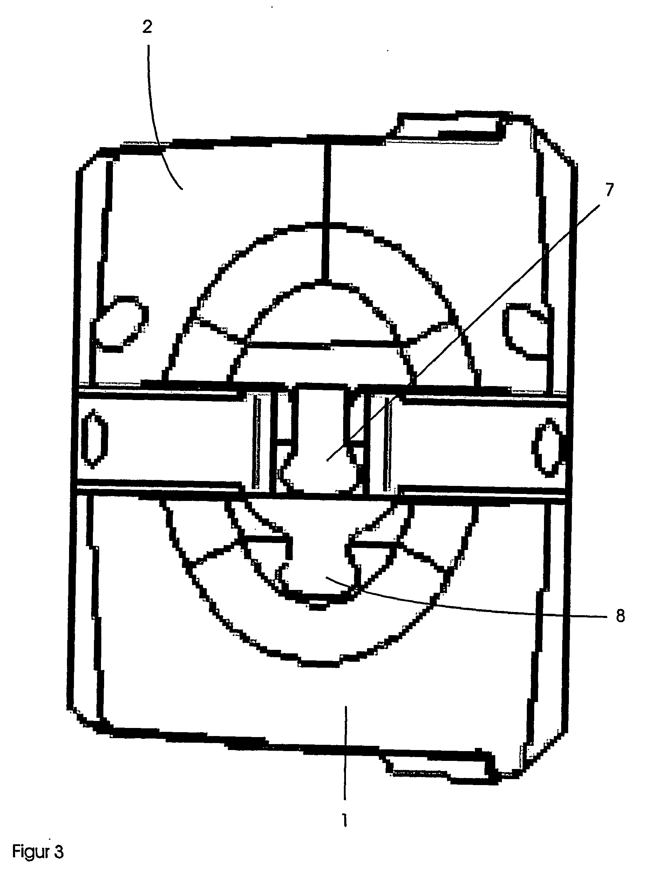

- Figur 3: eine Vorderansicht des in Figuren 1 und 2 gezeigten Implantates sowie
- Figur 4: eine perspektivische Darstellung des Implantates

Das erfindungsgemäße aufspreizbare Implantat gemäß Ausführungsbeispiel besteht aus zwei Schenkeln 1 und 2, die an einem Ende 3 miteinander verbunden sind.

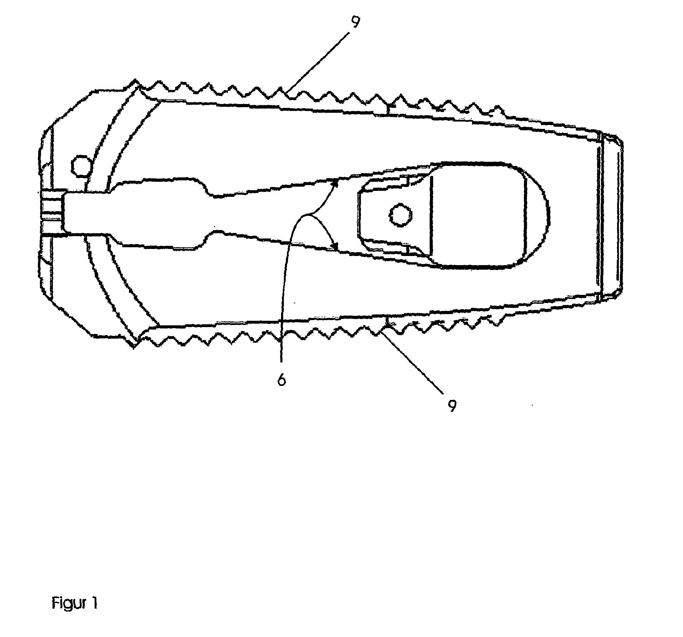

Die beiden Schenkel weisen wie in der Figur 1 ersichtlich eine Keilform auf.

Zwischen den beiden Schenkeln 1, 2 ist ein verschiebbares Spreizelement 4, insbesondere in Form eines Spreizblockes angeordnet. Das Spreizelement 4 ist in einer Nut 5 geführt, so dass eine translatorische Bewegung von einem Ende des Implantates zum anderen Ende möglich ist.

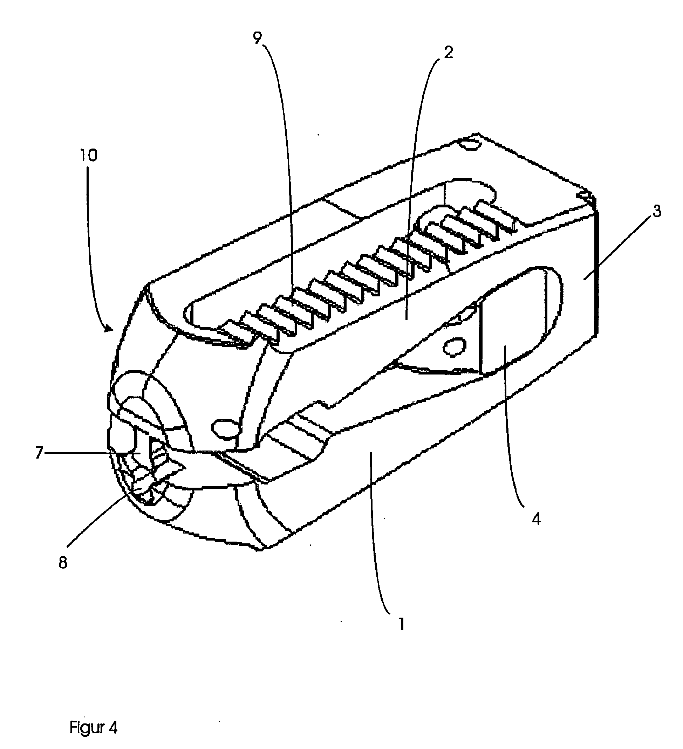

Durch Verschiebung des Spreizelementes 4 von der in Figur 4 gezeigten Position nach vorn, bewegen sich die beiden Schenkel 1, 2 von einander weg mit dem Ergebnis eines sich noch vergrößernden Keilwinkels im Vergleich zur Darstellung gemäß Figur 1.

Die durch die Schenkel 1, 2 gebildete Keilform bleibt also auch nach der Distraktion bezüglich des Keilwinkels mindestens erhalten oder unterliegt einer vorgegebenen Vergrößerung, wobei das Maß der Aufspreizung durch das Spreizelement 4 in Verbindung mit der konvergierenden Führung 6 an mindestens einer der Innenseiten der Schenkel 1, 2 bestimmt wird.

Im Bereich der freien Schenkelenden sind zum Fixieren der Schenkel 1, 2 in einer möglichst eng aneinanderliegenden Position kraft- und/oder formschlüssige, lösbare Arretierungsmittel angeordnet.

Die Arretierungsmittel bilden z. B. eine Snap-in-Verbindung.

Gemäß dem Ausführungsbeispiel ist an den gegenüberliegenden Flächen der freien Schenkelenden einerseits eine Rastnase 7 sowie andererseits eine hierzu komplementäre Rastnut 8 vorhanden.

In einem Abschnittsbereich der Außenflächen der Schenkel 1, 2 ist eine Verzahnung 9 vorhanden, welche für einen sicheren Halt des Implantates an der jeweiligen Wirbelkörperfläche Sorge trägt.

Beim Verschieben des Spreizelementes 4 zur Distraktion löst sich der Form- und/oder Kraftschluss zwischen Rastnut 8 und Rastnase 7 ohne weitere Maßnahmen.

Das freie Schenkelende 10 des Implantates weist eine Halbkugel-, sphärische oder Spitzform auf.

Wie aus den Darstellungen ersichtlich, ist das als Spreizblock ausgebildete Spreizelement 4 ausschließlich in der Lage eine Bewegung in eine zur Distraktion senkrechte Richtung auszuführen.

Weiterhin birgt das Implantat Bohrungen 11 zur Aufnahme von Röntgenkontraststiften, die insbesondere dann von Vorteil sind, wenn das Implantat aus einem nichtröntgenkontrastierenden Kunststoffmaterial besteht.

Das bevorzugte Kunststoffmaterial für das Implantat weist eine solche Restelastizität auf, dass eine Anpassung an Mikrobewegungen der jeweiligen Wirbelendplatte erfolgen kann. Selbstverständlich kann das Implantat auch aus einem elastischen metallischen Material bzw. entsprechenden Legierungen bestehen.

An den Ober- und Unterseiten der Schenkel 1, 2 vorgesehene Durchbrüche 12 dienen der Aufnahme von Knochensubstanz.

Unter Berücksichtigung der Darlegungen zum Ausführungsbeispiel der Erfindung ist bei dem vorgestellten Implantat ein optimal großes Verhältnis zwischen zusammengepressten und geöffneten Schenkeln des Implantates erreichbar, wobei auch im aufgespreizten Zustand eine konvexe äußere Geometrie der Schenkel erreicht wird.

Durch die Abrundung des Vorderendes des Implantates ist eine leichte Implantation in kleine Zwischenwirbelräume möglich und auch gleichzeitig eine optimale Anpassung an die ventrale Ringapophyse gegeben. Ebenso stellt sich ein Reserveraum, für das Einbringen von Spongiosa dar.

### Bezugszeichenliste:

- 1, 2: Schenkel
- 3: Verbindungsende
- 4: Spreizelement
- 5: Nut
- 6: konvergierende Führung
- 7: Rastnase
- 8: Rastnut
- 9: Verzahnung
- 10: freies Schenkelende
- 11: Bohrung
- 12: Durchbruch

## Patentansprüche

1. Aufspreizbares Implantat zur vorzugsweise dorsalen Anordnung zwischen den Wirbelkörpern der Wirbelsäule zum Zweck, diese mechanisch zu verbinden und eine knöcherne Durchbauung zu unterstützen, bestehend aus zwei, an einem Ende miteinander verbundenen Schenkeln (1, 2), welche eine Keilform bilden sowie mit einem zwischen den Schenkeln (1,2) angeordneten, insbesondere verschiebbaren Spreizelement (4) zur vertikalen Distraktion der Schenkel, wobei das Maß der Aufspreizung durch das Spreizelement (4) in Verbindung mit einer konvergierenden Führung (6) an mindestens einer der Innenseiten der Schenkel (1, 2) bestimmt wird
**dadurch gekennzeichnet, dass**
die durch die Schenkel (1, 2) gebildete Keilform auch nach der Distraktion bezüglich des Keilwinkels erhalten bleibt oder einer Vergrößerung unterliegt,wobei im Bereich der freien Schenkelenden zum Fixieren der Schenkel in einer möglichst eng aneinanderliegenden Position kraft- und/oder formschlüssige, lösbare Arretierungsmittel (7, 8) angeordnet sind.

2. Implantat nach Anspruch 1,
**dadurch gekennzeichnet, dass**
die Arretierungsmittel (7, 8) eine Snap-in-Verbindung bilden.

3. Implantat nach Anspruch 1 oder 2,
**dadurch gekennzeichnet, dass**
an den gegenüberliegenden Flächen der freien Schenkelenden einerseits eine Rastnase (7) sowie andererseits eine komplementäre Rastnut (8) ausgebildet ist.

4. Implantat nach einem der Ansprüche 1 bis 3,
**dadurch gekennzeichnet, dass**
beim Verschieben des Spreizelementes (4) zur Distraktion sich der Form- und/oder Kraftschluss zwischen den freien Schenkelenden selbsttätig löst.

5. Implantat nach einem der vorangegangenen Ansprüche,
**dadurch gekennzeichnet, dass**
das freie Schenkelende des Implantates eine Halbkugel-, sphärische oder Spitzform aufweist.

6. Implantat nach einem der vorangegangenen Ansprüche,
**dadurch gekennzeichnet, dass**
das Spreizelement (4) als Spreizblock ausgebildet ist, welcher ausschließlich eine Bewegung in eine zur Distraktion senkrechte Richtung ausführt.

7. Implantat nach einem der vorangegangenen Ansprüche,
**dadurch gekennzeichnet, dass**
dieses Nuten oder Bohrungen (11) für Röntgenkontrastmittel, insbesondere Röntgenkontraststifte aufweist.

8. Implantat nach Anspruch 1,
**dadurch gekennzeichnet, dass**
die Arretierungsmittel (7, 8) als integraler, einstückiger Bestandteil des Implantates ausgebildet sind.

9. Implantat nach einem der vorangegangenen Ansprüche,
**dadurch gekennzeichnet, dass**
dieses aus einem Kunststoffmaterial mit Restelastizität besteht.

10. Implantat nach einem der vorangegangenen Ansprüche,
**dadurch gekennzeichnet, dass**
die Ober- und Unterseiten der Schenkel Durchbrüche (12) zur Aufnahme von Knochensubstanz aufweisen.

## Claims

1. Expandable implant for a preferably dorsal arrangement between the vertebral bodies of the vertebral column, with the purpose of mechanically connecting the vertebral bodies and assisting in an osseous consolidation, comprising two legs (1, 2) connected to each other on one end and defining a wedge shape, and an expandable, in particular displaceable, element (4) arranged between the legs (1, 2) for the vertical distraction of the legs, wherein the expansion degree is determined by the expandable element (4) in association with a converging guide (6) on at least one of the inner sides of the legs (1, 2),
**characterized in that**
the wedge shape defined by the legs (1, 2) is maintained with respect to the wedge angle even after the distraction or is subject to an increase, wherein non-positive and/or positive separable locking means (7, 8) are disposed in the region of the free ends of the legs for fixing the legs in a position as closely spaced as possible.

2. Implant according to claim 1,
**characterized in that**
the locking means (7, 8) form a snap-in connection.

3. Implant according to claim 1 or 2,
**characterized in that**
a snap-in nose (7) on the one hand and a complementary snap-in groove (8) on the other hand are formed on the opposite surfaces of the free ends of the legs.

4. Implant according to one of claims 1 to 3,
**characterized in that**
the positive and/or non-positive connection between the free ends of the legs is automatically separated when the expandable element (4) is displaced for distraction.

5. Implant according to one of the preceding claims,
**characterized in that**
the free end of the leg of the implant is hemispherical, spherical or pointed.

6. Implant according to one of the preceding claims,
**characterized in that**
the expandable element (4) is formed as an expandable block which exclusively carries out a motion in a direction perpendicular with respect to the distraction.

7. Implant according to one of the preceding claims,
**characterized in that**
the implant comprises grooves or bores (11) for X-ray contrast agents, specifically X-ray contrast pins.

8. Implant according to claim 1,
**characterized in that**
the locking means (7, 8) are formed as an integral, one-piece component part of the implant.

9. Implant according to one of the preceding claims,
**characterized in that**
the implant is made of a plastic material having a residual elasticity.

10. Implant according to one of the preceding claims,
**characterized in that**
the upper and lower sides of the legs comprise through holes (12) for receiving bone substance.

## Revendications

1. Implant expansible destiné à être agencé de préférence en situation dorsale entre les corps vertébraux de la colonne vertébrale dans le but de relier ceux-ci de manière mécanique et de soutenir une consolidation osseuse, comprenant deux branches (1, 2) reliées l'une à l'autre à une extrémité, qui forment une conformation en coin, et comprenant un élément expansible (4) agencé entre les branches (1, 2) et en particulier déplaçable pour l'écartement vertical des branches, dans lequel la mesure de l'expansion par l'élément expansible (4) est déterminée en association avec un guidage convergent (6) sur l'une au moins des faces intérieures des branches (1, 2),
**caractérisé en ce que**
la conformation en coin formée par les branches (1, 2) demeure conservée également après l'écartement pour ce qui concerne l'angle du coin, ou subit une augmentation, et il est prévu des moyens d'arrêt libérables (7, 8) , à coopération de forces et/ou à coopération de formes, agencés dans la région des extrémités libres des branches pour la fixation des branches dans une position appliquée le plus étroitement possible l'une contre l'autre.

2. Implant selon la revendication 1,
**caractérisé en ce que** les moyens d'arrêt (7, 8) forment une liaison par encliquetage.

3. Implant selon la revendication 1 ou 2,
**caractérisé en ce qu'**un ergot d'enclenchement (7) d'une part et une rainure d'enclenchement complémentaire (8) d'autre part sont formés sur les surfaces opposées des extrémités libres des bras.

4. Implant selon l'une des revendications 1 à 3,
**caractérisé en ce que** lors du déplacement de l'élément expansible (4) pour l'écartement, la coopération de formes et/ou la coopération de forces entre les extrémités libres des branches se libère automatiquement.

5. Implant selon l'une des revendications précédentes,
**caractérisé en ce que** l'extrémité libre des bras de l'implant présente une forme hémisphérique, sphérique ou pointue.

6. Implant selon l'une des revendications précédentes,
**caractérisé en ce que** l'élément expansible (4) est réalisé sous forme de bloc expansible, qui exécute exclusivement un mouvement dans une direction perpendiculaire à l'écartement.

7. Implant selon l'une des revendications précédentes,
**caractérisé en ce que** celui-ci comprend des gorges ou des perçages (11) pour des moyens de contraste aux rayons X, en particulier des tiges de contraste aux rayons X.

8. Implant selon la revendication 1,
**caractérisé en ce que** les moyens d'arrêt (7, 8) sont réalisés sous forme de composant intégral d'un seul tenant de l'implant.

9. Implant selon l'une des revendications précédentes,
**caractérisé en ce que** celui-ci est en matière plastique avec une élasticité résiduelle.

10. Implant selon l'une des revendications précédentes,
**caractérisé en ce que** les faces supérieures et les faces inférieures des branches présentent des traversées (12) pour la réception de substance osseuse.
